(19) Europäisches Patentamt / European Patent Office / Office européen des brevets

(11) EP 4 524 580 A1

(12) EUROPEAN PATENT APPLICATION
published in accordance with Art. 153(4) EPC

(43) Date of publication:
19.03.2025 Bulletin 2025/12

(21) Application number: 23803249.4

(22) Date of filing: 23.03.2023

(51) International Patent Classification (IPC):
G01R 29/08 (2006.01)    G01N 22/00 (2006.01)
G01N 22/04 (2006.01)

(52) Cooperative Patent Classification (CPC):
G01N 22/00; G01N 22/04; G01R 29/08

(86) International application number:
PCT/JP2023/011413

(87) International publication number:
WO 2023/218766 (16.11.2023 Gazette 2023/46)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 13.05.2022 JP 2022079751

(71) Applicant: Sony Group Corporation
Tokyo 108-0075 (JP)

(72) Inventors:
• MIHOTA, Norihito
  Tokyo 108-0075 (JP)
• IIDA, Sachio
  Tokyo 108-0075 (JP)
• OISHI, Takahiro
  Tokyo 108-0075 (JP)

(74) Representative: D Young & Co LLP
3 Noble Street
London EC2V 7BQ (GB)

(54) **SENSOR DEVICE, INFORMATION PROCESSING DEVICE, INFORMATION PROCESSING METHOD, AND PROGRAM**

(57) A sensor apparatus according to an embodiment of the present technology includes an emitter, a detector, and a calculator. The emitter emits a plurality of electromagnetic waves of different frequencies to a measurement-target object. The detector detects the plurality of electromagnetic waves entering the detector through the measurement-target object. The calculator calculates a necessary number of times of emission for each of the plurality of electromagnetic waves on the basis of a result of the detection performed by the detector, the necessary number of times of emission being a certain number of times of emission that is necessary for measurement. This makes it possible to perform measurement efficiently with a high degree of accuracy when sensing is performed using an electromagnetic wave.

FIG.1

## Description

Technical Field

The present technology relates to a sensor apparatus, an information processing apparatus, an information processing method, and a program that can be applied to, for example, measurement of a moisture amount in soil.

Background Art

Patent Literature 1 discloses a sensor apparatus used to measure a moisture amount in soil. In this sensor apparatus, two probes between which transmission and reception of an electromagnetic wave are performed are inserted into soil such that the two probes are situated at a sufficiently large distance from each other. This enables a result of the measurement to be less affected by a space between the probe and the soil.

Citation List

Patent Literature

Patent Literature 1: WO 2018/221051

Disclosure of Invention

Technical Problem

There is a need for a technology that makes it possible to perform efficient measurement with a high degree of accuracy upon performing sensing by use of an electromagnetic wave.

In view of the circumstances described above, it is an object of the present technology to provide a sensor apparatus, an information processing apparatus, an information processing method, and a program that make it possible to perform efficient measurement with a high degree of accuracy upon performing sensing by use of an electromagnetic wave.

Solution to Problem

In order to achieve the object described above, a sensor apparatus according to an embodiment of the present technology includes an emitter, a detector, and a calculator.

The emitter emits a plurality of electromagnetic waves of different frequencies to a measurement-target object.

The detector detects the plurality of electromagnetic waves entering the detector through the measurement-target object.

The calculator calculates a necessary number of times of emission for each of the plurality of electromagnetic waves on the basis of a result of the detection performed by the detector, the necessary number of times of emission being a certain number of times of emission that is necessary for measurement.

In the sensor apparatus, a plurality of electromagnetic waves of different frequencies is emitted to a measurement-target object and is detected by the detector. Further, a necessary number of times of emission is calculated for each of the plurality of electromagnetic waves on the basis of a result of the detection of the electromagnetic wave. This makes it possible to perform measurement efficiently with a high degree of accuracy when sensing is performed using an electromagnetic wave.

The emitter may emit each of the plurality of electromagnetic waves the necessary number of times of emission that is calculated by the calculator. In this case, the sensor apparatus may further include an averaging processor that generates an average electromagnetic wave by averaging electromagnetic waves for the necessary number of times of emission that are detected by the detector, the averaging being performed for each of the plurality of electromagnetic waves.

The sensor apparatus may further include a generator that generates target-object information regarding the measurement-target object on the basis of the average electromagnetic wave generated by the averaging processor.

For each of the plurality of electromagnetic waves, the emitter may emit a calculation-use electromagnetic wave used to calculate the necessary number of times of emission. In this case, the calculator may calculate the necessary number of times of emission on the basis of a result of detection of the calculation-use electromagnetic wave that is performed by the detector.

On the basis of a signal-to-noise ratio of the calculation-use electromagnetic wave detected by the detector, the calculator may calculate, as the necessary number of times of emission, a certain number of times of emission with which a signal-to-noise ratio of the average electromagnetic wave generated by the averaging processor is greater than a specified threshold.

The calculator may calculate the necessary number of times of emission using the following formula:

[Math. 1]

$$N = 10^{\frac{T-R}{10}}$$

, where N represents the necessary number of times of emission, T (dB) represents the specified threshold regarding the signal-to-noise ratio of the average electromagnetic wave, and R (dB) represents the signal-to-noise ratio of the calculation-use electromagnetic wave detected by the detector.

The calculator may calculate the necessary number of times of emission using a lookup table in which a correspondence relationship between the signal-to-noise ratio of the calculation-use electromagnetic wave detected by the detector and the necessary number of times of emission is defined.

The emitter may emit the calculation-use electromagnetic waves in a frequency-based order with respect to the plurality of electromagnetic waves.

The emitter may emit the calculation-use electromagnetic waves in a random order with respect to the plurality of electromagnetic waves.

The emitter may control an intensity of the calculation-use electromagnetic wave for each of the plurality of electromagnetic waves.

The specified thresholds regarding the signal-to-noise ratios of the average electromagnetic waves for the plurality of electromagnetic waves may be set to an in-common value.

The specified threshold regarding the signal-to-noise ratio of the average electromagnetic wave may be set for each of the plurality of electromagnetic waves.

The specified threshold regarding the signal-to-noise ratio of the average electromagnetic wave may be set for each of the plurality of electromagnetic waves such that the necessary number of times of emission that is calculated by the calculator is not greater than an upper limit.

With respect to each of the plurality of electromagnetic waves, the calculator may set an upper limit of the necessary number of times of emission, and may calculate the necessary number of times of emission such that the necessary number of times of emission is not greater than the upper limit.

The calculator may calculate the necessary numbers of times of emission of the plurality of electromagnetic waves of the frequencies on the basis of a result of detection of a portion of the plurality of electromagnetic waves that is selected on a frequency basis.

The generator may calculate, as the target-object information, a moisture amount contained in the measurement-target object.

The measurement-target object may be soil. In this case, the generator may calculate, as the target-object information, a moisture amount contained in the soil.

An information processing apparatus according to an embodiment of the present technology includes an acquisition section and a calculator.

The acquisition section acquires a plurality of electromagnetic waves of different frequencies, each of the plurality of electromagnetic waves being emitted to a measurement-target object and detected by entering through the measurement-target object.

The calculator calculates a necessary number of times of emission for each of the plurality of electromagnetic waves acquired by the acquisition section, on the basis of each of the plurality of acquired electromagnetic waves, the necessary number of times of emission being a certain number of times of emission that is necessary for measurement.

An information processing method according to an embodiment of the present technology is an information processing method that is performed by a computer system, the information processing method including acquiring a plurality of electromagnetic waves of different frequencies, each of the plurality of electromagnetic waves being emitted to a measurement-target object and detected by entering through the measurement-target object.

A necessary number of times of emission is calculated for each of the plurality of acquired electromagnetic waves on the basis of the electromagnetic wave, the necessary number of times of emission being a certain number of times of emission that is necessary for measurement.

A program according to an embodiment of the present technology causes a computer system to perform a process including:

acquiring a plurality of electromagnetic waves of different frequencies, each of the plurality of electromagnetic waves being emitted to a measurement-target object and detected by entering through the measurement-target object; and
calculating a necessary number of times of emission for each of the plurality of acquired electromagnetic waves on the basis of the electromagnetic wave, the necessary number of times of emission being a certain number of times of emission that is necessary for measurement.

Brief Description of Drawings

[Fig. 1] Fig. 1 schematically illustrates an example of a configuration of a sensor apparatus according to an embodiment of the present technology.
[Fig. 2] Fig. 2 is a flowchart illustrating a processing example of averaging processing.
[Fig. 3] Fig. 3 is a flowchart illustrating the processing example of the averaging processing.
[Fig. 4] Fig. 4 schematically illustrates a level of, an SNR of, and the number of times of emission of an electromagnetic wave.
[Fig. 5] Fig. 5 schematically illustrates a frequency of a transmitted electromagnetic wave.
[Fig. 6] Fig. 6 schematically illustrates a level, an SNR, and a necessary number of times of emission of an electromagnetic wave in a sensor apparatus according to a comparative example.
[Fig. 7] Fig. 7 schematically illustrates a transmission level of a calculation-use electromagnetic wave.
[Fig. 8] Fig. 8 schematically illustrates an SNR of a calculation-use electromagnetic wave and a necessary SNR of an average electromagnetic wave.

[Fig. 9] Fig. 9 schematically illustrates an SNR of a calculation-use electromagnetic wave and a necessary SNR of an average electromagnetic wave.

[Fig. 10] Fig. 10 is a flowchart illustrating a processing example of the averaging processing.

[Fig. 11] Fig. 11 is a flowchart illustrating the processing example of the averaging processing.

[Fig. 12] Fig. 12 is a block diagram illustrating an example of a hardware configuration of a computer by which a measurement unit and an information processing apparatus can be implemented.

Mode(s) for Carrying Out the Invention

Embodiments according to the present technology will now be described below with reference to the drawings.

[Sensor Apparatus]

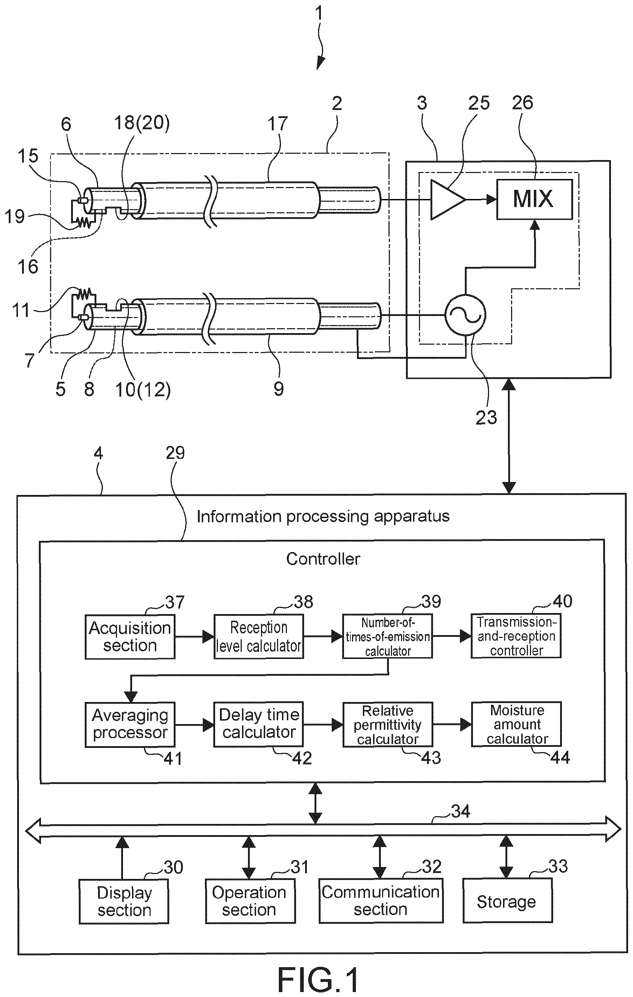

Fig. 1 schematically illustrates an example of a configuration of a sensor apparatus according to an embodiment of the present technology. In the present embodiment, the sensor apparatus 1 calculates a moisture amount contained in soil. For example, the sensor apparatus 1 is used to measure a moisture amount in soil where crops will grow.

The sensor apparatus 1 transmits an electromagnetic wave into soil, and the sensor apparatus 1 detects the electromagnetic wave passing through the soil. Further, a moisture amount contained in the soil is calculated on the basis of a result of the detection of the electromagnetic wave. Moreover, a moisture amount contained in any object other than the soil may be calculated. Further, a parameter other than a moisture amount may be measurable.

The sensor apparatus 1 includes a sensor head 2, a measurement unit 3, and an information processing apparatus 4. The sensor head 2 includes a transmission probe 5 and a reception probe 6. The transmission probe 5 includes a core wire portion 7, a shield portion 8, a sleeve 9, an antenna section 10, and an end resistance 11.

The core wire portion 7 is an electric wire used to carry an electric signal. For example, a copper wire is used as the core wire portion 7.

The shield portion 8 is a member that is a base body of the transmission probe 5. The shield portion 8 has a cylindrical shape. Further, the shield portion 8 includes, for example, a copper pipe or a mesh of copper wires. The core wire portion 7 is embedded in the shield portion 8 along a central axis of the shield portion 8.

A surface of the shield portion 8 is covered with a protection layer (not illustrated). The protection layer is a member used to insulate the surface of the shield portion 8, and is made of, for example, an insulating material. Further, in particular, a portion of the surface of the shield portion 8 that is situated near a front end of the shield portion 8 is covered with an electromagnetic-wave-transmissive protection member (not illustrated). Of course, portions of the surface of the shield portion 8 that are respectively covered with a protection layer and a protection member are not limited.

The sleeve 9 is a member that covers the surface of the shield portion 8. The sleeve 9 includes an electromagnetic wave absorptive material. Ferrite is primarily used as the electromagnetic wave absorptive material. However, another material, such as sendust or permalloy, that exhibits a high magnetic permeability may be used. In the present embodiment, the shield portion 8 covered with a protection layer and a protection member is further covered with the sleeve 9. For example, the side surface of the shield portion 8 except for portions that are situated near the front end and near a back end of the shield portion 8 is covered with the sleeve 9. Note that the sleeve 9 may be omitted as appropriate if necessary.

The antenna section 10 is an antenna used to emit an electromagnetic wave to the outside of the transmission probe 5. For example, the antenna section 10 is provided as a tiny antenna to a portion of the shield portion 8 that is situated near the front end of the shield portion 8 within the shield portion 8.

Further, an opening 12 is provided to a portion of the side surface of the shield portion 8 that is situated near the front end of the shield portion 8. The opening 12 is formed as an opening having a shape of an ellipse that has a major axis extending in parallel with the central axis of the shield portion 8. The length of the major axis may be set as appropriate according to a wavelength of an electromagnetic wave emitted by the antenna section 10. When, for example, the electromagnetic wave has a wavelength of from 500 MHz to 8 GHz, the length of the major axis is set to be a length of from 5 mm to 15 mm. Of course, the major axis corresponding to the opening 12 is not limited. Further, the shape of the opening 12 is also not limited to an ellipse, and any shape such as a rectangle or a circle may be adopted.

In the present embodiment, when an electromagnetic wave is emitted by the antenna section 10, the emitted electromagnetic wave leaks out of the transmission probe 5 through the opening 12. Accordingly, an electromagnetic wave is emitted to the outside of the transmission probe 5 (into soil).

The end resistance 11 is electrically connected to the core wire portion 7 and to the side surface of the shield portion 8. The end resistance 11 is made of, for example, a material exhibiting a certain level of electric resistance.

The transmission probe 5 is vertically inserted into soil. Further, a thickness of the transmission probe 5 is set to be a thickness of, for example, from about 2 mm to about 6 mm. Such a setting of the thickness makes it possible to easily perform insertion into soil. Of course, a specific shape of the transmission probe 5 is not limited.

Further, specific shapes, materials, and the like of the core wire portion 7, the shield portion 8, the sleeve 9, the antenna section 10, the end resistance 11, the protection layer, and the protection member are also not limited. The transmission probe 5 corresponds to an embodiment of an emitter according to the present technology.

The reception probe 6 includes a core wire portion 15, a shield portion 16, a sleeve 17, an antenna section 18, and an end resistance 19. Further, an opening 20 is provided to a portion of the side surface of the shield portion 16 that is situated near a front end of the shield portion 16. The core wire portion 15, the shield portion 16, the sleeve 17, the antenna section 18, the end resistance 19, and the opening 20 respectively have configurations respectively similar to the configurations of the core wire portion 7, shield portion 8, sleeve 9, antenna section 10, end resistance 11, and opening 12 included in the transmission probe 5. Thus, descriptions thereof are omitted. The reception probe 6 corresponds to an embodiment of a detector according to the present technology.

The reception probe 6 is vertically inserted into soil to be situated at a specified distance from the transmission probe 5 such that the respective openings 12 and 20 face each other. The distance between the transmission probe 5 and the reception probe 6 is not limited. If the distance is greater than 100 mm, there may be an increase in the attenuation of an electromagnetic wave that propagates through soil. This may result in difficulty in obtaining a sufficient detection intensity. Further, if the distance is less than 20 mm, the electromagnetic wave may be greatly affected by spaces formed near the transmission probe 5 and the reception probe **6.** This may result in difficulty in measuring a moisture amount correctly. Thus, the distance between the transmission probe 5 and the reception probe 6 is set to be a distance of, for example, from about 20 mm to about 100 mm.

The measurement unit 3 includes a signal generator 23, an amplifier 25, and a mixer 26.

The signal generator 23 generates a transmission signal. In the present embodiment, the signal generator 23 generates a continuous wave as the transmission signal. Without being limited thereto, the signal generator 23 may generate, for example, a pulse wave (a pulse signal) as the transmission signal. The transmission signal generated by the signal generator 23 is input to the transmission probe 5. Further, the antenna section 10 of the transmission probe 5 emits an electromagnetic wave dependent on the acquired transmission signal.

When an electromagnetic wave is detected by the antenna section 18 of the reception probe 6, a reception signal dependent on the detected electromagnetic wave is output to the amplifier 25. The amplifier 25 amplifies the reception signal acquired from the reception probe 6. The reception signal amplified by the amplifier 25 is output to the mixer 26.

The mixer 26 is a mechanism used to mix signals. In the present embodiment, the transmission signal generated by the signal generator 23 is split by a phase shifter (not illustrated) into two transmission signals that are 90 degrees out of phase. Further, the mixer 26 acquires the two transmission signals that are 90 degrees out of phase, and the amplified reception signal. Then, the acquired signals are mixed to generate a measurement signal. The generated measurement signal is transmitted to the information processing apparatus 4.

The sensor head 2, the measurement unit 3, and the information processing apparatus 4 may be provided in a state of being physically connected to each other by wired cables. Further, the sensor head 2, the measurement unit 3, and the information processing apparatus 4 may be provided on a single substrate. Alternatively, the sensor head 2, the measurement unit 3, and the information processing apparatus 4 may be capable of communicating with each other in a state of being physically separated from each other. In this case, for example, the sensor head 2 and the measurement unit 3 each include a communication section, and the communication sections enable transmission and reception of, for example, a transmission signal, a reception signal, and a measurement signal.

The information processing apparatus 4 includes a controller 29, a display section 30, an operation section 31, a communication section 32, and a storage 33. The controller 29, the display section 30, the operation section 31, the communication section 32, and the storage 33 are connected to each other through a bus 34. The respective blocks may be connected to each other using, for example, a communication network or an unstandardized unique communication approach instead of the use of the bus 34.

The display section 21 is a display device using, for example, liquid crystal or electroluminescence (EL), and, for example, various images and various graphical user interfaces (GUIs) are displayed on the display section 21.

Examples of the operation section 31 include a keyboard, a pointing device, a touchscreen, and other operation apparatuses. When the operation section 31 includes a touchscreen, the touchscreen may be integrated with the display section 21. For example, a user who uses the sensor apparatus 1 can perform setting related to an operation of the sensor apparatus 1 using the operation section 31.

The communication section 32 is a communication module used to communicate with another device through a network such as a local area network (LAN) or a wide area network (WAN). The communication section 32 may be provided with a communication module, such as Bluetooth (registered trademark), that is used to perform near-field communication. Further, the communication section 32 may be provided with communication equipment such as a modem and a router. In the present embodiment, the communication section 32 causes the information processing apparatus 4 to communicate with the measurement unit 3. Moreover, the information processing apparatus 4 may be capable of communicating with any apparatus other than the measurement unit 3.

The storage 33 is a storage device such as a nonvolatile memory, and, for example, an HDD or an SSD is used. Moreover, any non-transitory computer-readable storage medium may be used as the storage 33. The storage 33 stores therein a control program used to control an operation of the overall information processing apparatus 4. A method for installing the control program on the information processing apparatus 4 is not limited. For example, the installation may be performed through various recording media, or the installation of the program may be performed through, for example, the Internet.

The controller 29 includes hardware, such as a processor including a CPU, a GPU, and a DSP; a memory including a ROM and a RAM; and a storage device including an HDD, that is necessary for a configuration of a computer. For example, an information processing method according to the present technology is performed by the CPU loading, into the RAM, a program according to the present technology that is recorded in, for example, the ROM in advance and executing the program. For example, a programmable logic device (PLD) such as a field programmable gate array (FPGA), or another device such as an application specific integrated circuit (ASIC) may be used as the controller 29.

In the present embodiment, an acquisition section 37, a reception level calculator 38, a number-of-times-of-emission calculator 39, a transmission-and-reception controller 40, an averaging processor 41, a delay time calculator 42, a relative permittivity calculator 43, and a moisture amount calculator 44 are implemented as functional blocks by the CPU of the controller 29 executing the program according to the present technology (such as an application program). Note that, in order to implement each functional block, dedicated hardware such as an integrated circuit (IC) may be used as appropriate.

The acquisition section 37 acquires a measurement signal from the measurement unit 3. In the present embodiment, the measurement signal includes information regarding an electromagnetic wave detected by the reception probe 6. Thus, it can also be said that the acquisition section 37 acquires an electromagnetic wave. The electromagnetic wave is acquired by the acquisition section 37 through the communication section 32. Further, the acquired electromagnetic wave is stored in the storage 33.

The reception level calculator 38 calculates a reception level of an electromagnetic wave acquired by the acquisition section 37. In the present embodiment, the reception level calculator 38 calculates an intensity of the electromagnetic wave as the reception level. Further, a signal-to-noise ratio (SNR) of the electromagnetic wave is calculated on the basis of the calculated intensity.

The number-of-times-of-emission calculator 39 calculates the number of times of emission of the electromagnetic wave on the basis of the SNR of the electromagnetic wave that is calculated by the reception level calculator 38. A specific method for calculating the number of times of emission will be described in detail later. The reception level calculator 38 and the number-of-times-of-emission calculator 39 correspond to an embodiment of a calculator according to the present technology.

The transmission-and-reception controller 40 controls emission of an electromagnetic wave that is performed by the transmission probe 5 and detection of the electromagnetic wave that is performed by the reception probe 6. For example, an operation of the signal generator 23 of the measurement unit 3 is controlled through the communication section 32 to control emission of an electromagnetic wave that is performed by the transmission probe 5. Moreover, any method may be used to control emission and detection of an electromagnetic wave.

In the present embodiment, the transmission-and-reception controller 40 transmits, to the signal generator 23, an instruction to emit an electromagnetic wave the number of times of emission that is calculated by the number-of-times-of-emission calculator 39. Then, the transmission probe 5 emits the electromagnetic wave the number of times of emission. Further, the reception probe 6 detects the electromagnetic wave the number of times of emission. The electromagnetic waves for the number of times of emission are acquired by the acquisition section 37 and stored in the storage 33.

The averaging processor 41 acquires electromagnetic waves for the number of times of emission and stored in the storage 33. Further, the respective electromagnetic waves are averaged to generate an average electromagnetic wave. Electromagnetic waves are averaged by, for example, combining waveforms. Of course, electromagnetic waves may be averaged by any other method.

The delay time calculator 42 calculates a propagation delay time of an electromagnetic wave between the transmission probe 5 and the reception probe 6 on the basis of an average electromagnetic wave. Typically, the propagation delay time of an electromagnetic wave is a difference that the propagation time of the electromagnetic wave in a medium has with the propagation time of the electromagnetic wave in air. A propagation delay time of an electromagnetic wave depends on the relative permittivity of a transmission path, and is proportional to the square root of the relative permittivity of a medium. In general, the relative permittivity of soil is about 1 to 10, and varies depending on a moisture amount. Thus, the moisture amount in the medium can be indirectly measured if it is possible to measure a propagation delay time.

The relative permittivity calculator 43 calculates the relative permittivity of soil on the basis of a propagation delay time of an electromagnetic wave that is calculated by the delay time calculator 42.

The moisture amount calculator 44 calculates a moisture amount contained in soil, on the basis of the relative permittivity of the soil that is calculated by the relative permittivity calculator 43. The delay time calculator 42, the relative permittivity calculator 43, and the moisture amount calculator 44 correspond to an embodiment of a generator according to the present technology.

[Averaging Processing]

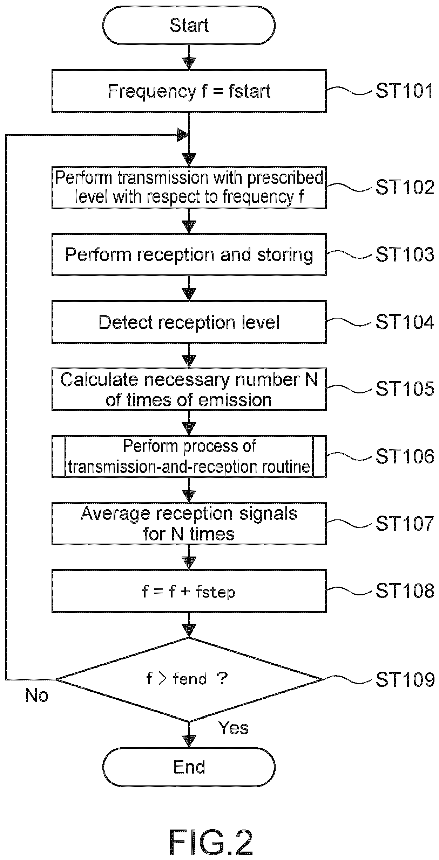

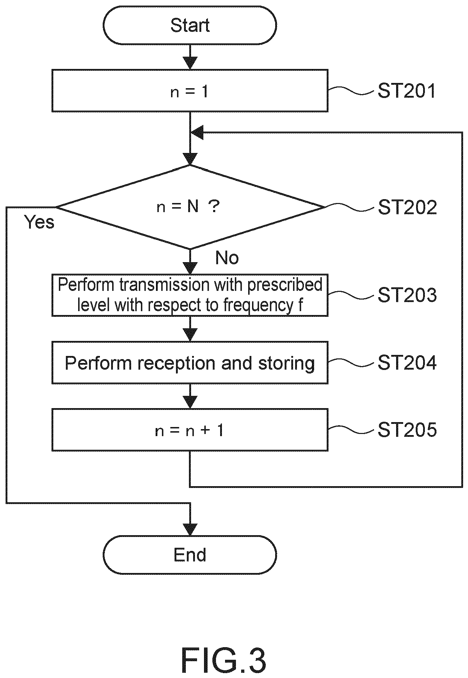

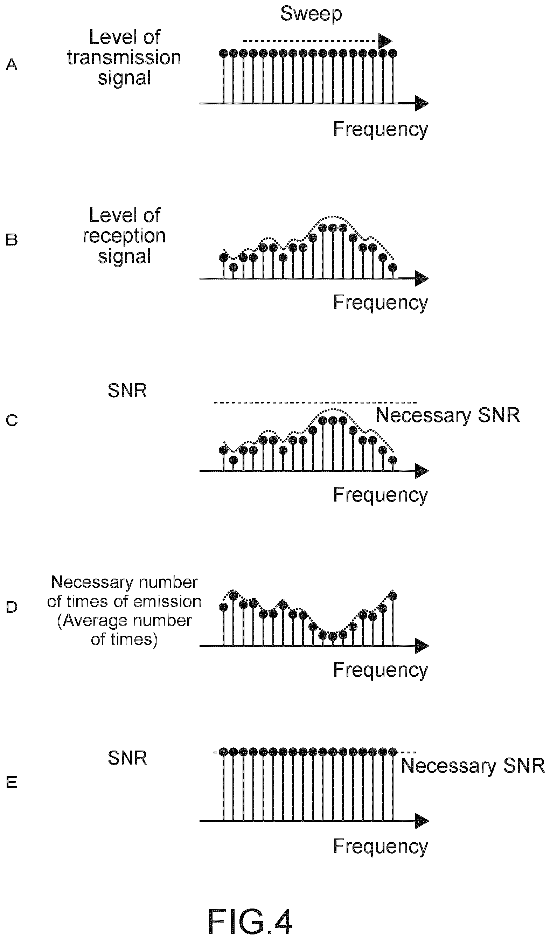

Processing of averaging electromagnetic waves is described. Figs. 2 and 3 are flowcharts illustrating a processing example of averaging processing. Fig. 4 schematically illustrates a level of, an SNR of, and the number of times of emission of an electromagnetic wave.

In the present embodiment, the transmission probe 5 emits a plurality of electromagnetic waves of different frequencies to soil. For example, a plurality of electromagnetic waves of different frequencies in which a difference between adjacent frequencies is equal, is emitted. Specifically, frequency spacing is 0.05 GHz (50 MHz), with a lower limit of 1.00 GHz and an upper limit of 6.00 GHz such as "1.00 GHz, 1.05 GHz, 1.10 GHz, ···, 5.95 GHz, and 6.00 GHz". A plurality of electromagnetic waves satisfying such a condition is emitted. In this case, 101 kinds of electromagnetic waves are emitted in total.

In the following description, the 101 kinds of electromagnetic waves described above are assumed to be emitted in order to facilitate the understanding of the description. Of course, the lower limit and upper limit of and frequency spacing for frequencies of a plurality of electromagnetic waves emitted by the transmission probe 5 are not limited. Further, a plurality of electromagnetic waves with unequal frequency spacing may be emitted.

Further, emission of an electromagnetic wave that is performed by the transmission probe 5 may be referred to as "transmission of an electromagnetic wave". Likewise, detection of an electromagnetic wave that is performed by the reception probe 6 may be referred to as "reception of an electromagnetic wave". In other words, expressions such as "the number of times of emission" and "the number of times of transmission" may be used without being particularly distinguished.

A frequency of an electromagnetic wave transmitted by the transmission probe 5 is set to a lower limit (Step 101). Specifically, a parameter "fstart" representing the lower limit is substituted for a parameter "f" representing the frequency. In the present embodiment, the lower limit of the frequency for the 101 kinds of electromagnetic waves is 1.00 GHz. Thus, 1.00 GHz, which is fstart, is substituted for f.

The transmission probe 5 emits a calculation-use electromagnetic wave at the frequency f (Step 102). The calculation-use electromagnetic wave is an electromagnetic wave used to calculate a necessary number of times of emission. Specific details of the necessary number of times of emission, and a method for calculating the necessary number of times of emission using the calculation-use electromagnetic wave will be described in detail later.

In the present embodiment, a prescribed level that is a specified level is set in advance as a transmission level of a calculation-use electromagnetic wave. The calculation-use electromagnetic wave at the frequency f (= 1.00 GHz) is transmitted by the transmission probe 5 with the prescribed level. Note that a specific value of the prescribed level is not limited.

A of Fig. 4 schematically illustrates a transmission level of a calculation-use electromagnetic wave transmitted in Step 102. A vertical axis of a bar chart illustrated in A of Fig. 4 represents the transmission level of the calculation-use electromagnetic wave, and a horizontal axis of the bar chart represents a frequency of the calculation-use electromagnetic wave. A leftmost bar in the bar chart in A of Fig. 4 represents a transmission level and a frequency of a calculation-use electromagnetic wave at the frequency of 1.00 GHz.

Further, in the present embodiment, after a series of processing performed with respect to an electromagnetic wave at the frequency of 1.00 GHz is completed, similar processing is performed with respect to an electromagnetic wave at the frequency of 1.05 GHz, which is the next frequency. The second bar from the left in A of Fig. 4 represents a transmission level and a frequency of a calculation-use electromagnetic wave at the frequency of 1.05 GHz. Likewise, the respective bars also represent other calculation-use electromagnetic waves. Note that calculation-use electromagnetic waves at 101 kinds of frequencies are transmitted in total in the present embodiment. However, A of Fig. 4 illustrates eighteen bars in order to simplify representation.

The calculation-use electromagnetic wave is received by the reception probe 6 and stored (Step 103). In the present embodiment, a plurality of electromagnetic waves of different frequencies that enters the reception probe 6 through soil is detected by the reception probe **6.** Specifically, the calculation-use electromagnetic wave at the frequency of 1.00 GHz that is transmitted in Step 102 propagates through soil and is received by the reception probe 6. Note that similar processing is performed with respect to each of calculation-use electromagnetic waves of other frequencies later and the calculation-use electromagnetic wave is received by the reception probe 6.

Note that the case in which an electromagnetic wave enters the reception probe 6 through soil includes not only the case in which the electromagnetic wave propagates through the soil, but also any other cases in which the electromagnetic wave enters the reception probe 6 by being affected by the soil such as by being reflected off the soil or by being refracted by the soil.

Further, the calculation-use electromagnetic wave at the frequency of 1.00 GHz that is received by the reception probe 6 is acquired by the acquisition section 37 and stored in the storage 33.

The reception level calculator 38 detects a reception level of the calculation-use electromagnetic wave (Step 104). Specifically, the calculation-use electromagnetic wave at the frequency of 1.00 GHz that is stored in the storage 33 is acquired by the reception level calculator 38, and the reception level is detected by the reception level calculator 38.

B of Fig. 4 schematically illustrates a reception level of a calculation-use electromagnetic wave. A vertical axis of a bar chart illustrated in B of Fig. 4 represents the reception level of the calculation-use electromagnetic wave, and a horizontal axis of the bar chart represents a frequency of the calculation-use electromagnetic wave. A leftmost bar in the bar chart represents a reception level and a frequency of a calculation-use electromagnetic wave at the frequency of 1.00 GHz.

Further, the reception level calculator 38 calculates an SNR of the calculation-use electromagnetic wave at the frequency of 1.00 GHz. C of Fig. 4 schematically illustrates an SNR of the calculation-use electromagnetic wave received by the reception probe 6 in Step 103. A vertical axis of a bar chart illustrated in C of Fig. 4 represents the SNR of the calculation-use electromagnetic wave, and a horizontal axis of the bar chart represents a frequency of the calculation-use electromagnetic wave.

In the present embodiment, noise included in the received calculation-use electromagnetic wave is assumed to be white noise in order to facilitate the understanding of the description. Of course, the present technology can also be applied to the case in which noise exhibits a frequency distribution. When noise is white noise, the charts of the reception level of and the SNR of a calculation-use electromagnetic wave have the same shape, as illustrated in B and C of Fig. 4.

In the present embodiment, a moisture amount in soil is calculated on the basis of an electromagnetic wave. There is a good possibility that a sufficiently accurate measurement result will not be obtained when the electromagnetic wave exhibits a low SNR (that is, the percentage of noise is high). For example, when an electromagnetic wave at the frequency of 1.00 GHz for one time that is received by the reception probe 6 exhibits a low SNR, and when a moisture amount is calculated on the basis of only the electromagnetic wave at the frequency of 1.00 GHz for one time, there is a good possibility of calculating an erroneous moisture amount.

In order to avoid such a situation, averaging processing is performed by the averaging processor 41. In general, an electromagnetic wave at a certain frequency is emitted multiple times, and the detected electromagnetic waves are averaged. This makes it possible to obtain an electromagnetic wave exhibiting a high SNR. For example, an electromagnetic wave obtained by detecting electromagnetic waves at the frequency of 1.00 GHz twice and by averaging the detected electromagnetic waves, exhibits a higher SNR than the electromagnetic wave at the frequency of 1.00 GHz, which is detected once. Likewise, when the number of electromagnetic waves used for averaging is increased, such as detecting the electromagnetic wave three times, four times, and the like, this makes it possible to further increase the SNR of an average electromagnetic wave.

In the present embodiment, a necessary signal-to-noise ratio (a necessary SNR) of an average electromagnetic wave is set in advance. In consideration of, for example, a desired measurement accuracy, the necessary SNR is determined by, for example, a user who uses the sensor apparatus 1. Specifically, when a moisture amount is desired to be calculated with a high degree of accuracy, the necessary SNR is set to a relatively large value. When, conversely, there is no need for a high degree of accuracy, the necessary SNR is set to be a relatively small value. Of course, a specific method for setting a necessary SNR is not limited.

In C of Fig. 4, a set value of a necessary SNR of an average electromagnetic wave is indicated using a dashed line. In the present embodiment, specified thresholds regarding SNRs of average electromagnetic waves for a plurality of electromagnetic waves are set to an in-common value. Here, the specified threshold regarding an SNR of an average electromagnetic wave is the necessary SNR. In other words, the necessary SNR is set to a fixed value regardless of frequency. Without being limited thereto, different necessary SNRs may be set for respective frequencies.

As illustrated in C of Fig. 4, the SNR of a calculation-use electromagnetic wave at the frequency of 1.00 GHz that is received by the reception probe 6 has not reached a value of the necessary SNR. In other words, there is a good possibility that a sufficiently accurate measurement result will not be obtained if the calculation-use electromagnetic wave is used to calculate a moisture amount with no change. Thus, there is a need to increase the SNR by emitting the electromagnetic wave at the frequency of 1.00 GHz multiple times, by detecting the electromagnetic waves, and by performing averaging processing with respect to the detected electromagnetic waves.

The number-of-times-of-emission calculator 39 calculates the number of times of emission of an electromagnetic wave used to perform averaging processing (Step 105). In the present embodiment, the number-of-times-of-emission calculator 39 calculates a necessary number of times of emission for each of electromagnetic waves at a plurality of frequencies on the basis of a result of detection performed by the reception probe 6, where the necessary number of times of emission is a certain number of times of emission that is necessary for measurement.

Specifically, the number of times of emission with which an SNR of an average electromagnetic wave generated by the averaging processor 41 is greater than a specified threshold, is calculated as a necessary number of times of emission. In other words, the number of times of emission (the number of electromagnetic waves used to perform averaging) with which an SNR of an average electromagnetic wave is greater than a necessary SNR, is calculated as a necessary number of times of emission. Of course, a parameter other than the necessary SNR may be used as the specified threshold.

In the present embodiment, the number-of-times-of-emission calculator 39 calculates a necessary number of times of emission on the basis of a result of detection of a calculation-use electromagnetic that is performed by the reception probe 6. Specifically, the number-of-times-of-emission calculator 39 calculates a necessary number of times of emission on the basis of an SNR of a calculation-use electromagnetic wave detected by the reception probe 6.

Specifically, for example, the number-of-times-of-emission calculator 39 calculates a necessary number of times of emission using the following formula:

[Math. 1]

$$N = 10^{\frac{T-R}{10}}$$

, where N represents a necessary number of times of emission, T (dB) represents a necessary SNR of an average electromagnetic wave, and R (dB) represents an SNR of a calculation-use electromagnetic wave detected by the reception probe 6.

When, for example, a necessary SNR of an average electromagnetic wave is 70 (dB) and an SNR of the calculation-use electromagnetic wave detected by the reception probe 6 is 60 (dB), a necessary number N of times of emission is ten. When a value of the SNR of the calculation-use electromagnetic wave is relatively close to a value of the necessary SNR, a relatively small number of times is calculated as the necessary number of times of emission. On the other hand, when the value of the SNR of the calculation-use electromagnetic wave is relatively distant from the value of the necessary SNR, a relatively large number of times is calculated as the necessary number of times of emission.

Alternatively, the number-of-times-of-emission calculator 39 may calculate a necessary number of times of emission using a lookup table in which a correspondence relationship between an SNR of a calculation-use electromagnetic wave detected by the reception probe 6 and the necessary number of times of emission is defined. For example, a relationship represented by "necessary SNR: 70, SNR of calculation-use electromagnetic wave: 60, necessary number of times of emission: 10" is defined in a lookup table, and the necessary number of times of emission is output, with a necessary SNR and an SNR of a calculation-use electromagnetic wave being used as input.

Moreover, a specific method for calculating a necessary number of times of emission is not limited. For example, the necessary number of times of emission may be calculated on the basis of a parameter other than the SNRs. Further, the necessary number of times of emission may be calculated without using a calculation-use electromagnetic wave.

D of Fig. 4 schematically illustrates a necessary number of times of emission that is calculated by the number-of-times-of-emission calculator 39. A vertical axis of a bar chart illustrated in D of Fig. 4 represents the necessary number of times of emission of an electromagnetic wave, and a horizontal axis of the bar chart represents a frequency of the electromagnetic wave. Note that, in C and D of Fig. 4, logarithmic values are taken on the respective vertical axes. Thus, when, for example, the necessary number of times of emission is calculated using the (Math. 1) formula, the respective charts have inverse shapes in the up-and-down direction, as illustrated in C and D of Fig. 4. In the following description, ten is assumed to be calculated as the necessary number N of times of emission of an electromagnetic wave at the frequency of 1.00 GHz, in order to facilitate the understanding of the description.

A process of a transmission-and-reception routine is performed by the transmission probe 5 and the reception probe 6 (Step 106).

The transmission-and-reception controller 40 sets a variable n that represents a cumulative number of times of emission, such that n = 1 (Step 201). Next, the transmission-and-reception controller 40 determines whether the cumulative number n of times of emission is equal to the necessary number N of times of emission (Step 202). When the process of Step 202 is performed for the first time, the cumulative number of times of emission is not equal to the necessary number N of times of emission since N = 10 and n = 1. Thus, it is determined to be No in Step 202.

When the cumulative number n of times of emission is not equal to the necessary number N of times of emission (No in Step 202), an electromagnetic wave is transmitted (Step 203). Specifically, the transmission probe 5 transmits an electromagnetic wave at the frequency of 1.00 GHz with a prescribed level once. In the present embodiment, a level exhibiting a value that is equal to a value of a prescribed level used to transmit a calculation-use electromagnetic wave is used as the prescribed level used to transmit the electromagnetic wave at the frequency of 1.00 GHz.

The electromagnetic wave is received and stored (Step 204). Specifically, the electromagnetic wave at the frequency of 1.00 GHz is received by the reception probe 6 and stored in the storage 33.

One is added to the cumulative number n of times of emission (Step 205). When, for example, the process of Step 205 is performed for the first time, one is added to the cumulative number n of times of emission, where n = 1 + 1 = 2.

Next, it is determined again whether the cumulative number n of times of emission is equal to the necessary number N of times of emission (Step 202). When the process of Step 202 is performed for the second time, the cumulative number n of times of emission is two. Thus, the cumulative number n of times of emission, which is two, is also not equal to the necessary number N of times of emission, which is ten. Thus, it is determined to be No in Step 202.

As described above, a series of processes of Steps 202 to 205 is performed until it is determined to be Yes in Step 202. It is determined to be Yes in Step 202 for the first time when the cumulative number n of times of emission is ten. Thus, the processes of emission, detection, and storing of an electromagnetic wave in Steps 202 to 205 are performed nine times in total. Thus, the storage 33 stores therein the electromagnetic waves at the frequency of 1.00 GHz for nine times.

As described above, a calculation-use electromagnetic wave is emitted once in the process of Step 102, and an electromagnetic wave is transmitted nine times in the process of Step 106. In other words, the transmission probe 5 emits a plurality of electromagnetic waves of different frequencies a necessary number of times of emission. Specifically, an electromagnetic wave at the frequency of 1.00 GHz is transmitted ten times, which is the necessary number of times of emission.

Averaging processing is performed with respect to the electromagnetic waves (Step 107). With respect to each of the plurality of electromagnetic waves of different frequencies, the averaging processor 41 averages electromagnetic waves for the necessary number of times of emission that are detected by the reception probe 6. Accordingly, an average electromagnetic wave is generated. Specifically, first, the averaging processor 41 acquires electromagnetic waves at the frequency of 1.00 GHz for nine times that are stored in the storage 33. Further, the averaging processor 41 acquires a calculation-use electromagnetic wave at the frequency of 1.00 GHz that is stored in the storage 33. Furthermore, the averaging processor 41 averages the electromagnetic waves for nine times and the calculation-use electromagnetic wave to generate an average electromagnetic wave. In other words, an average electromagnetic wave is generated from the electromagnetic waves for ten times.

As described above, electromagnetic waves for a certain number of times that is one less than the necessary number of times of emission are stored in the process of Step 106, and a calculation-use electromagnetic wave for one time is stored in the process of Step 103. Accordingly, the electromagnetic waves for the necessary number of times of emission can be obtained in total. Of course, electromagnetic waves for the necessary number of times of emission may be stored in the process of Step 106, and the calculation-use electromagnetic wave does not necessarily have to be used to perform averaging processing.

E of Fig. 4 schematically illustrates an SNR of an average electromagnetic wave generated by the averaging processor 41 in Step 107. A vertical axis of a bar chart illustrated in E of Fig. 4 represents the SNR of the average electromagnetic wave, and a horizontal axis of the bar chart represents a frequency of the average electromagnetic wave. An SNR of an average electromagnetic wave at the frequency of 1.00 GHz (a leftmost bar) has reached exactly a value of a necessary SNR.

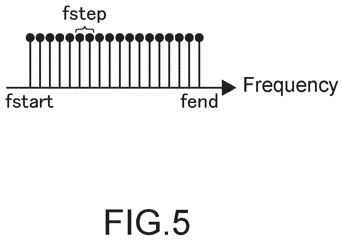

A parameter fstep is added to the parameter f representing a frequency (Step 108). Fig. 5 schematically illustrates a frequency of a transmitted electromagnetic wave. As illustrated in Fig. 5, fstep is a parameter that represents spacing for frequencies of transmitted electromagnetic waves. In the present embodiment, a plurality of electromagnetic waves at frequencies with spacing of 0.05 GHz is transmitted. Thus, fstep = 0.05 GHz. In other words, when the process of Step 108 is performed for the first time, fstep, which is 0.05 GHz, is added to f, which is 1.00 GHz. As a result, f = 1.05 GHz.

It is determined whether the parameter f representing the frequency is greater than a parameter fend (Step 109). fend is a parameter that represents an upper limit of a frequency of a transmitted electromagnetic wave. In the present embodiment, the upper limit of the frequency is 6.00 GHz. Thus, fend = 6.00 GHz. When the process of Step 109 is performed for the first time, f = 1.05 GHz, and fend = 6.00 GHz. Thus, it is determined to be No in Step 109.

When the parameter f representing the frequency is not greater than fend (No in Step 109), a series of the processes of Steps 102 to 109 is performed again. In other words, first, similar processing including transmission and reception of a calculation-use electromagnetic wave, calculation of a necessary number of times of emission, transmission and reception of electromagnetic waves for the necessary number of times of emission, and averaging is performed with respect to an electromagnetic wave at the frequency of 1.05 GHz. Such processing is continuously performed until f is determined to be greater than fend in Step 109 (that is, until "f = 6.05 GHz" is satisfied). Thus, similar processing is performed with respect to each of the frequencies of 1.10 GHz, 1.15 GHz, ···, and 6.00 GHz.

In the present embodiment, a calculation-use electromagnetic wave at a frequency other than the frequency of 1.00 GHz is also transmitted with a certain prescribed level exhibiting the same value as the prescribed level used for the frequency of 1.00 GHz, as illustrated in A of Fig. 4. Of course, a transmission level of a calculation-use electromagnetic wave may differ depending on the frequency.

Further, a reception level of a received calculation-use electromagnetic wave may exhibit a value that differs depending on the frequency, as illustrated in B of Fig. 4. Specifically, a reception level of an electromagnetic wave exhibits a value that differs depending on the frequency due to, for example, a frequency response exhibited by the electromagnetic wave. Thus, each of an SNR of a calculation-use electromagnetic wave and a necessary number of times of emission of the calculation-use electromagnetic wave also exhibits a value that differs depending on the frequency, as illustrated in C and D of Fig. 4.

Further, SNRs of a plurality of average electromagnetic waves of different frequencies respectively have reached exactly values of necessary SNRs, as illustrated in E of Fig. 4.

[Generation of Target-Object Information]

Generation of target-object information is described. In the present embodiment, the delay time calculator 42, the relative permittivity calculator 43, and the moisture amount calculator 44 generate target-object information regarding a measurement-target object on the basis of an average electromagnetic wave generated by the averaging processor 41. Specifically, the target-object information is generated on the basis of all of average electromagnetic waves at the frequencies of from 1.00 GHz to 6.00 GHz. Processing of generating the target-object information is performed after a series of the processes of Steps 101 to 109 and 201 to 205 is performed.

The measurement-target object is an object that is to be measured by the sensor apparatus 1. In the present embodiment, the delay time calculator 42, the relative permittivity calculator 43, and the moisture amount calculator 44 calculate, as the target-object information, a moisture amount contained in soil. In other words, the measurement-target object is soil in the present embodiment. In this case, the sensor apparatus 1 may be referred to as, for example, a moisture sensor or a soil moisture sensor.

Of course, the measurement-target object is not limited, and may be any object other than soil. For example, a moisture amount contained in, for example, food may be calculable. Further, generated target-object information is also not limited to the moisture amount, and any parameter related to the measurement-target object may be generated.

A calculated moisture amount may be displayed on the display section 30 after the calculation of the moisture amount. Alternatively, various parameters such as a reception level and an SNR of a calculation-use electromagnetic wave, a necessary number of times of emission, an SNR of an average electromagnetic wave, a propagation delay time, and the relative permittivity may be displayed. Further, a necessary SNR and others may be settable through the operation section 31.

In the sensor apparatus 1 according to the present embodiment, a plurality of electromagnetic waves of different frequencies is emitted to soil and is detected by the reception probe 6, as described above. Further, a necessary number of times of emission is calculated for each of the plurality of electromagnetic waves on the basis of a result of the detection of the electromagnetic wave. This makes it possible to perform measurement efficiently with a high degree of accuracy when sensing is performed using an electromagnetic wave.

There is a need to use an electromagnetic wave compliant with the Radio Law when sensing is performed using a soil moisture sensor of a radiofrequency (RF)-transmission type. This results in restricting an output level of an electromagnetic wave. Thus, an SNR of a reception signal exhibits a relatively small value. Therefore, there is a need to perform processing of increasing an SNR by repeatedly transmitting an electromagnetic wave and by averaging the transmitted electromagnetic waves.

However, the repeated transmission of an electromagnetic wave results in taking a long time to perform sensing, and thus in increasing power consumption. Therefore, there are demands for reducing the measurement time and power consumption.

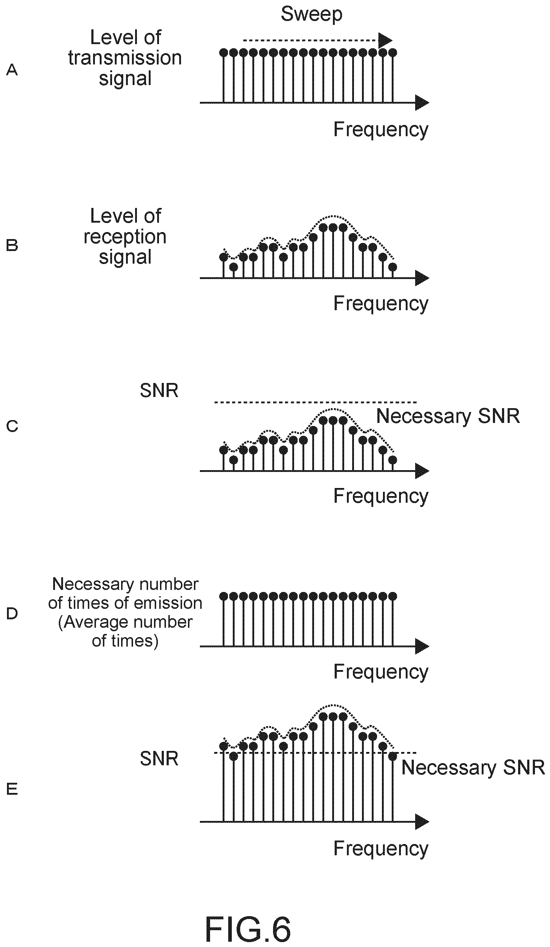

Fig. 6 schematically illustrates a level, an SNR, and a necessary number of times of emission of an electromagnetic wave in a sensor apparatus according to a comparative example. B and C of Fig. 6 respectively illustrate a level and an SNR of a received electromagnetic wave when the electromagnetic wave is transmitted with a prescribed level, as illustrated in A of Fig. 6.

In the sensor apparatus according to the comparative example, necessary numbers of times of emission for respective frequencies are set equally, as illustrated in D of Fig. 6. The necessary number of times of emission is set in advance such that SNRs of average electromagnetic waves at all of the frequencies are each greater than a necessary SNR. For example, an SNR of an average electromagnetic wave is inconsistent due to, for example, a surrounding environment. The number of times is set in consideration of some safety margin in order not for the SNR of the average electromagnetic wave to be less than the necessary SNR even when there is the inconsistency. Note that a calculation-use electromagnetic wave is not transmitted in the sensor apparatus according to the comparative example, compared to the case of the sensor apparatus 1 according to the present technology.

Consequently, the SNRs of the respective average electromagnetic waves each exceed the necessary SNR, as illustrated in E of Fig. 6, although it is essentially sufficient if each of them has reached exactly a value of the necessary SNR. The reason is that the numbers of times of transmission for all of the frequencies are equally determined. In other words, it can be said that the necessary numbers of times of emission of electromagnetic waves at the respective frequencies are also calculated in excess (overcalculated, compared to an essentially necessary number of times).

On the other hand, in the sensor apparatus 1 according to the present technology, a necessary number of times of emission is calculated for each frequency such that an SNR of an average electromagnetic wave reaches exactly a value of a necessary SNR. In other words, the necessary numbers of times of emission for all of the frequencies are not calculated equally, which is different from the sensor apparatus according to the comparative example. Thus, the necessary numbers of times of emission for the respective frequencies are different from each other, as illustrated in D of Fig. 4. As illustrated in D of Fig. 4 and D of Fig. 6, a total of the necessary numbers of times of emission for all of the frequencies is less in the sensor apparatus 1 according to the present technology than in the sensor apparatus according to the comparative example.

This makes it possible to reduce the time necessary to transmit and receive an electromagnetic wave. This further makes it possible to reduce power consumption. This makes it possible to, for example, perform sensing efficiently while keeping a high degree of accuracy in measurement.

Further, in the present embodiment, the averaging processor 41 averages electromagnetic waves for a necessary number of times of emission to generate an average electromagnetic wave. This makes it possible to perform measurement accurately using an average electromagnetic wave.

Further, the transmission probe 5 emits a calculation-use electromagnetic wave used to calculate a necessary number of times of emission, and the reception level calculator 38 and the number-of-times-of-emission calculator 39 calculate the necessary number of times of emission on the basis of a result of detection of the calculation-use electromagnetic wave that is performed by the reception probe 6. This results in accurately calculating the necessary number of times of emission.

Furthermore, on the basis of an SNR of a calculation-use electromagnetic wave detected by the reception probe 6, the reception level calculator 38 and the number-of-times-of-emission calculator 39 calculate, as the necessary number of times of emission, a certain number of times of emission with which an SNR of an average electromagnetic wave is greater than a specified threshold. This results in accurately calculating the necessary number of times of emission.

Further, the reception level calculator 38 and the number-of-times-of-emission calculator 39 calculate the necessary number of times of emission using the formula. This results in accurately calculating the necessary number of times of emission.

Furthermore, the reception level calculator 38 and the number-of-times-of-emission calculator 39 calculate the necessary number of times of emission using a lookup table in which a correspondence relationship between an SNR of a calculation-use electromagnetic wave and the necessary number of times of emission is defined. This results in accurately calculating the necessary number of times of emission.

Further, the delay time calculator 42, the relative permittivity calculator 43, and the moisture amount calculator 44 generate target-object information regarding a measurement-target object on the basis of an average electromagnetic wave. This makes it possible to accurately generate information regarding the measurement-target object.

Particularly in the present embodiment, the delay time calculator 42, the relative permittivity calculator 43, and the moisture amount calculator 44 calculate a moisture amount contained in soil on the basis of an average electromagnetic wave. This makes it possible to accurately calculate the moisture amount contained in the soil.

<Other Embodiments>

The present technology is not limited to the embodiments described above, and can achieve various other embodiments.

The order of frequencies at which calculation-use electromagnetic waves are emitted is not limited, and any order may be adopted. For example, the transmission probe 5 emits calculation-use electromagnetic waves in a frequency-based order with respect to a plurality of electromagnetic waves. Specifically, calculation-use electromagnetic waves are emitted in ascending order of frequencies. Alternatively, calculation-use electromagnetic waves may be emitted in descending order of frequencies. This makes it possible to perform processing efficiently in consideration of, for example, frequency response. Moreover, how to set the frequency-based order is not limited.

Further, for example, the transmission probe 5 may emit calculation-use electromagnetic waves in a random order with respect to a plurality of electromagnetic waves. This makes it possible to perform processing efficiently.

In the example illustrated in A of Fig. 4, the transmission level of a calculation-use electromagnetic wave is fixed regardless of frequency. However, the transmission level does not necessarily have to be fixed. Specifically, the transmission probe 5 controls an intensity of a calculation-use electromagnetic wave for each one of a plurality of electromagnetic waves.

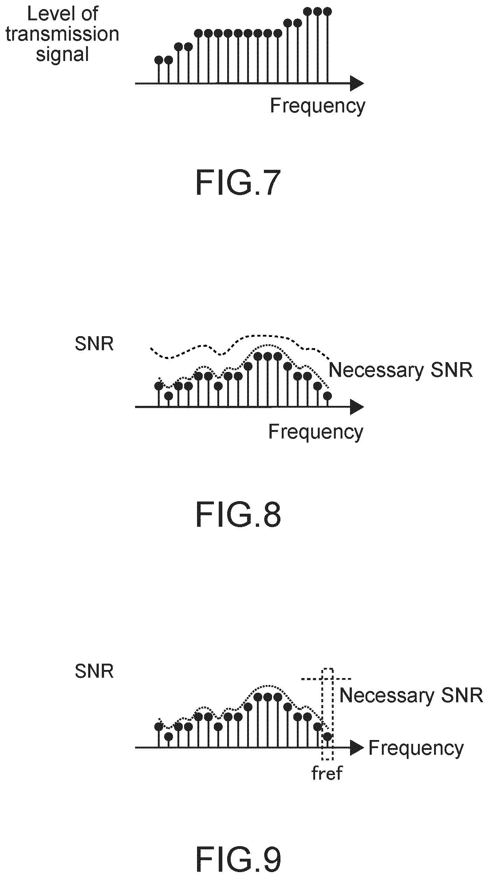

Fig. 7 schematically illustrates a transmission level of a calculation-use electromagnetic wave. In this example, the transmission level is set for each frequency in advance such that calculation-use electromagnetic waves are emitted with different transmission levels for respective frequencies. For example, the transmission level is set to be relatively low for a low frequency band. On the other hand, the transmission level is set to be relatively high for a high frequency band. For example, a user who uses the sensor apparatus 1 sets, in advance, a transmission level of a calculation-use electromagnetic wave for each frequency. This makes it possible to, for example, emit a calculation-use electromagnetic wave with a suitable transmission level according to a frequency response. This results in accurately calculating a necessary number of times of emission.

In the example illustrated in C of Fig. 4, the necessary SNR of an average electromagnetic wave is fixed regardless of frequency. However, the necessary SNR does not necessarily have to be fixed. Specifically, the necessary SNR of an average electromagnetic wave is set for each one of a plurality of electromagnetic waves. Further, the necessary SNR of an average electromagnetic wave is set for each of the plurality of electromagnetic waves such that the necessary number of times of emission is not greater than an upper limit.

Fig. 8 schematically illustrates an SNR of a calculation-use electromagnetic wave and a necessary SNR of an average electromagnetic wave. In this example, the necessary SNRs are set to different values for respective frequencies. Specifically, the necessary SNR is also set to be relatively low for a frequency at which the SNR of a calculation-use electromagnetic wave is relatively low. On the other hand, the necessary SNR is also set to be relatively high for a frequency at which the SNR of a calculation-use electromagnetic wave is relatively high.

For example, when, with respect to a certain frequency, the SNR of a calculation-use electromagnetic wave is low in excess, compared to the necessary SNR, an unrealistic necessary number of times of emission (such as N = 10000) may be calculated. The necessary SNR is also set to a small value when the SNR of a calculation-use electromagnetic wave is relatively low. This results in selecting a reasonable number of times of emission as a necessary number of times of emission, and an unrealistic necessary number of times of emission is no longer calculated.

In this case, the necessary SNRs are set for the respective frequencies such that a necessary number of times of emission is not greater than an upper limit. When, for example, the upper limit is set such that "N = 100", the necessary number of times of emission is first calculated on the basis of the SNR of a calculation-use electromagnetic wave. When the calculated necessary number of times of emission is not greater than 100, the necessary SNR set in advance is used with no change. On the other hand, when the calculated necessary number of times of emission is greater than 100, the necessary SNR set in advance is corrected such that the necessary number of times of emission is 100. Of course, how to set the necessary SNR is not limited. For example, the necessary SNR may be set using another method that is not based on the SNR of a calculation-use electromagnetic wave.

Alternatively, not the necessary SNR but the necessary number of times of emission itself may be corrected. Specifically, with respect to each one of a plurality of electromagnetic waves, the reception level calculator 38 and the number-of-times-of-emission calculator 39 set an upper limit of the necessary number of times of emission, and calculate the necessary number of times of emission such that the necessary number of times of emission is not greater than the upper limit.

In this case, the necessary number of times of emission is first calculated on the basis of the SNR of a calculation-use electromagnetic wave. When the calculated necessary number of times of emission is not greater than 100, the calculated necessary number of times of emission is used with no change. On the other hand, when the calculated necessary number of times of emission is greater than 100, the calculated necessary number of times of emission is corrected to be 100. As described above, the processing of correcting a necessary number of times of emission may be performed without changing a necessary SNR internally.

Necessary numbers of times of emission for all of the frequencies may be calculated on the basis of SNRs of calculation-use electromagnetic waves corresponding to a portion of all of the frequencies. Specifically, the reception level calculator 38 and the number-of-times-of-emission calculator 39 calculate necessary numbers of times of emission of electromagnetic waves at respective frequencies on the basis of a result of detection of a portion of the electromagnetic waves that is selected on a frequency basis.

When a frequency response of an electromagnetic wave is considered, this may make it possible to estimate an SNR of a calculation-use electromagnetic wave at a certain frequency on the basis of an SNR of a calculation-use electromagnetic wave at another frequency. For example, there is a possibility of exhibiting the characteristics in that an SNR of a calculation-use electromagnetic wave remains unchanged in a low frequency band but is reduced in a high frequency band. In this case, SNRs of calculation-use electromagnetic waves at all of the frequencies can be estimated by, for example, finding out an SNR of a calculation-use electromagnetic wave at a frequency that is most affected by a moisture amount.

Fig. 9 schematically illustrates an SNR of a calculation-use electromagnetic wave and a necessary SNR of an average electromagnetic wave. In this example, a necessary number of times of emission of an electromagnetic wave at a frequency fref is calculated in order to calculate necessary numbers of times of emission for all of the frequencies. In other words, an electromagnetic wave at the frequency fref is only selected as a portion of electromagnetic waves that is selected on a frequency basis. Of course, the selection is not limited thereto, and a plurality of electromagnetic waves, or electromagnetic waves at frequencies in a specified range may be selected as the portion of the electromagnetic waves that is selected on a frequency basis.

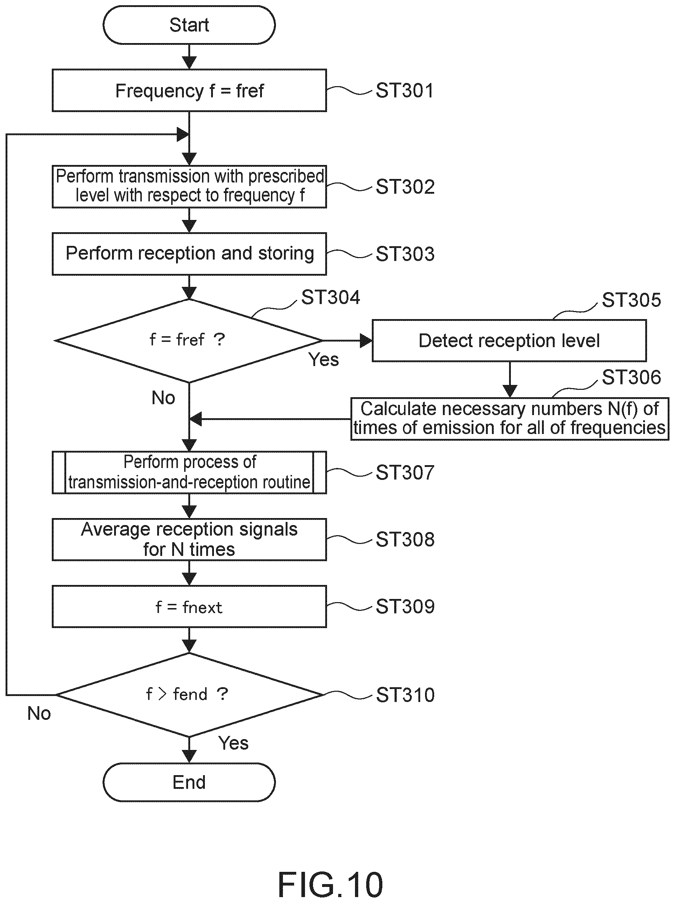

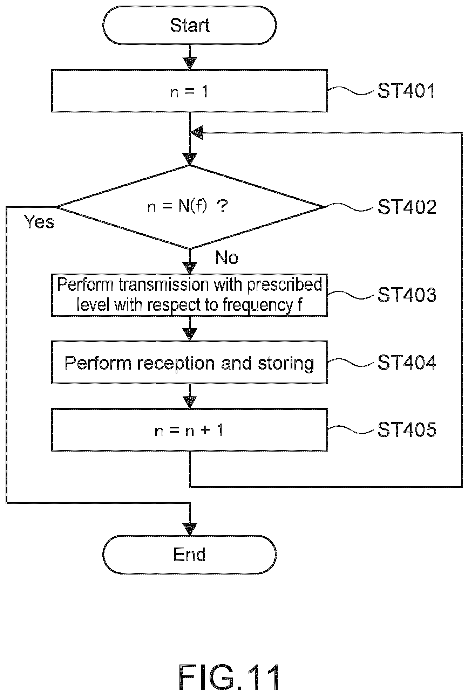

Figs. 10 and 11 are flowcharts illustrating a processing example of the averaging processing. In the processing example illustrated in Figs. 10 and 11, all of the necessary numbers of times of emission are calculated on the basis of a necessary number of times of emission of an electromagnetic wave at the frequency fref to perform averaging processing. In the following description, fref is assumed to be 1.10 GHz in order to facilitate the understanding of the description. Further, descriptions of contents of the processes of Steps 301 to 310 and 401 to 405 that are similar to contents of the processes of Steps 101 to 109 and 201 to 205 are omitted as appropriate.

In the processes of Steps 301 to 303, fref is set to be the parameter f representing the frequency, and a calculation-use electromagnetic wave is transmitted, received, and stored at the frequency fref.

It is determined whether f = fref (Step 304). When the process of Step 304 is performed for the first time, f = fref. Thus, it is determined to be Yes in Step 304.

When it has been determined that f = fref (Yes in Step 304), a reception level of the calculation-use electromagnetic wave at the frequency fref is detected (Step 305). Further, an SNR of the calculation-use electromagnetic wave at the frequency fref is calculated.

Necessary numbers of times of emission for all of the frequencies are calculated (Step 306). First, SNRs of calculation-use electromagnetic waves at the respective frequencies are calculated. The SNRs of the calculation-use electromagnetic waves at the respective frequencies are calculated using the prediction approach described above on the basis of the SNR of the calculation-use electromagnetic wave at the frequency fref. Further, a lookup table in which a correspondence relationship between an SNR of a calculation-use electromagnetic wave at the frequency fref and each of SNRs of calculation-use electromagnetic waves at respective frequencies is defined, is provided in advance, and the SNRs of the calculation-use electromagnetic waves at the respective frequencies may be calculated using the lookup table.

Further, a necessary number of times of emission for each frequency is calculated on the basis of the calculated SNR of the calculation-use electromagnetic wave at the frequency. Specifically, for example, the SNR of the calculation-use electromagnetic wave at each frequency is substituted for the (Math. 1) formula to calculate the necessary number of times of emission for the frequency.

The calculated necessary number of times of emission for each frequency is substituted for a variable N(f). When, for example, 50 is calculated as a necessary number of times of emission of an electromagnetic wave at the frequency of 3.00 GHz, N (3.00 GHz) = 50.

In the processes of Steps 307 and 308, an electromagnetic wave at the frequency fref is transmitted and received a certain number of times that is one less than a necessary number N(fref) of times of emission to perform averaging processing.

fnext is substituted for the parameter f (Step 309). Here, fnext is a parameter that is determined by a current value of f. Specifically, fnext = fstart when f = fref. Further, when "f = fref" does not hold, fnext = f + fstep (note that fstep is further added when fnext = fref).

When the process of Step 309 is performed for the first time, f = fref. Thus, fnext = fstart. Therefore, fstart (1.00 GHz) is substituted for f.

It is determined whether f is greater than fend (Step 310). It is determined to be No in Step 310 since f = 1.00 GHz and fend = 6.00 GHz.

First, the averaging processing is performed for an electromagnetic wave at the frequency fref (1.10 GHz), as described above. Likewise, next, the averaging processing is performed for an electromagnetic wave at the frequency f = 1.00 GHz. Here, 1.05 GHz is substituted for f in the process of Step 309, and it is determined to be No in Step 310.

Further, processing when f = 1.05 GHz is performed. Here, fnext = f + fstep = 1.10 GHz in Step 309, and fnext = fref. Thus, fstep is further added, and fnext = 1.15 GHz.

Thus, processing for the frequency of 1.15 GHz is performed after the processing when f = 1.05 GHz is performed. Thereafter, processing for the frequency of 1.20 GHz, processing for the frequency of 1.25 GHz, ..., and processing for the frequency of 6.00 GHz are performed. In other words, with respect to all of the averaging processing, processing for the frequency fref (1.10 GHz) is performed first, and thereafter, processes are performed in ascending order of frequencies (except for fref, which is to be skipped).

This results in there being no need to transmit and receive calculation-use electromagnetic waves for frequencies other than the frequency fref, and in being able to reduce the time and power necessary for measurement.

A portion of or all of the functions of the sensor head 2 or measurement unit 3 illustrated in Fig. 1 may be included in the information processing apparatus 4. Alternatively, a portable information processing apparatus 4 may be used. In other words, the sensor apparatus 1 may be implemented by a plurality of computers or a single computer.

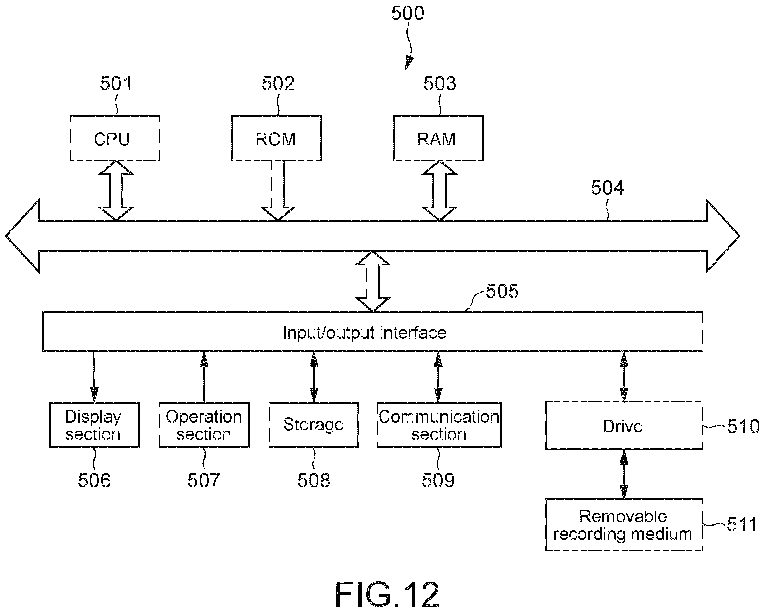

Fig. 12 is a block diagram illustrating an example of a hardware configuration of a computer 500 by which the measurement unit 3 and the information processing apparatus 4 can be implemented. The computer 500 includes a CPU 501, a ROM 502, a RAM 503, an input/output interface 505, and a bus 504 through which these components are connected to each other. A display section 506, an input section 507, a storage 508, a communication section 509, a drive 510, and the like are connected to the input/output interface 505.

The display section 506 is a display device using, for example, liquid crystal or EL. Examples of the input section 507 include a keyboard, a pointing device, a touchscreen, and other operation apparatuses. When the input section 507 includes a touchscreen, the touchscreen may be integrated with the display section 506. The storage 508 is a nonvolatile storage device, and examples of the storage 508 include an HDD, a flash memory, and other solid-state memories. The drive 510 is a device that can drive a removable recording medium 511 such as an optical recording medium or a magnetic recording tape. The communication section 509 is a modem, a router, or another communication apparatus that can be connected to, for example, a LAN or a WAN and is used to communicate with another device. The communication section 509 may perform communication wirelessly or by wire. The communication section 509 is often used in a state of being separate from the computer 500.

Information processing performed by the computer 500 having the hardware configuration described above is performed by software stored in, for example, the storage 508 or the ROM 502, and hardware resources of the computer 500 working cooperatively. Specifically, the information processing method according to the present technology is performed by loading, into the RAM 503, a program included in the software and stored in the ROM 502 or the like and executing the program.

For example, the program is installed on the computer 500 through the removable recording medium 511. Alternatively, the program may be installed on the computer 500 through, for example, a global network. Moreover, any non-transitory storage medium that is readable by the computer 500 may be used.

The information processing method according to the present technology may be executed and the sensor apparatus or information processing apparatus according to the present technology may be implemented by a plurality of computers working cooperatively, the plurality of computers being a plurality of computers connected through, for example, a network to be capable of communicating with each other. In other words, the information processing method according to the present technology can be executed not only in a computer system that includes a single computer, but also in a computer system in which a plurality of computers operates cooperatively.

Note that, in the present disclosure, the system refers to a set of components (such as apparatuses and modules (parts)) and it does not matter whether all of the components are in a single housing. Thus, a plurality of apparatuses accommodated in separate housings and connected to each other through a network, and a single apparatus in which a plurality of modules is accommodated in a single housing are both the system.

The execution of the information processing method according to the present technology by the computer system includes, for example, both the case in which the transmission and reception of a calculation-use electromagnetic wave, the calculation of a reception level, the calculation of an SNR, the calculation of a necessary number of times of emission, the averaging processing, the generation of target-object information, and the like are executed by a single computer; and the case in which the respective processes are executed by different computers. Further, the execution of the respective processes by a specified computer includes causing another computer to execute a portion of or all of the processes and acquiring a result of it. In other words, the information processing method according to the present technology is also applicable to a configuration of cloud computing in which a single function is shared and cooperatively processed by a plurality of apparatuses through a network.

The respective configurations of the sensor apparatus, the sensor head, the measurement unit, and the information processing apparatus; the respective processing flows; and the like described with reference to the respective figures are merely embodiments, and any modifications may be made thereto without departing from the spirit of the present technology. In other words, for example, any other configurations or algorithms for purpose of practicing the present technology may be adopted.

When wording such as "substantially" is used in the present disclosure, such wording is merely used to facilitate the understanding of the description, and whether the wording such as "substantially" is used has no particular significance. In other words, in the present disclosure, expressions, such as "center", "middle", "uniform", "equal", "similar", "orthogonal", "parallel", "symmetric", "extend", "axial direction", "columnar", "cylindrical", "ring-shaped", "annular", "circular", "rectangular", and "elliptic" that define, for example, a shape, a size, a positional relationship, and a state respectively include, in concept, expressions such as "substantially the center/substantial center", "substantially the middle/substantially middle", "substantially uniform", "substantially equal", "substantially similar", "substantially orthogonal", "substantially parallel", "substantially symmetric", "substantially extend", "substantially axial direction", "substantially columnar", "substantially cylindrical", "substantially ring-shaped", "substantially annular", "substantially circular", "substantially rectangular", and "substantially elliptic". For example, the expressions such as "center", "middle", "uniform", "equal", "similar", "orthogonal", "parallel", "symmetric", "extend", "axial direction", "columnar", "cylindrical", "ring-shaped", "annular", "circular", "rectangular", and "elliptic" also respectively include states within specified ranges (such as a range of +/-10%), with expressions such as "exactly the center/-exact center", "exactly the middle/exactly middle", "exactly uniform", "exactly equal", "exactly similar", "completely orthogonal", "completely parallel", "completely symmetric", "completely extend", "fully axial direction", "perfectly columnar", "perfectly cylindrical", "perfectly ring-shaped", "perfectly annular", "perfectly circular", "perfectly rectangular", and "perfectly elliptic" being respectively used as references. Thus, an expression that does not include the wording such as "substantially" can also include, in concept, a possible expression including the wording such as "substantially". Conversely, a state expressed using the expression including the wording such as "substantially" may include a state of "exactly/exact", "completely", "fully", or "perfectly".

In the present disclosure, an expression using "-er than" such as "being larger than A" and "being smaller than A" comprehensively includes, in concept, an expression that includes "being equal to A" and an expression that does not include "being equal to A". For example, "being larger than A" is not limited to the expression that does not include "being equal to A", and also includes "being equal to or greater than A". Further, "being smaller than A" is not limited to "being less than A", and also includes "being equal to or less than A". When the present technology is carried out, it is sufficient if a specific setting or the like is adopted as appropriate from expressions included in "being larger than A" and expressions included in "being smaller than A", in order to provide the effects described above.

At least two of the features of the present technology described above can also be combined. In other words, the various features described in the respective embodiments may be combined discretionarily regardless of the embodiments. Further, the various effects described above are not limitative but are merely illustrative, and other effects may be provided.

Note that the present technology may also take the following configurations.

(1) A sensor apparatus, including:

an emitter that emits a plurality of electromagnetic waves of different frequencies to a measurement-target object;
a detector that detects the plurality of electromagnetic waves entering the detector through the measurement-target object; and
a calculator that calculates a necessary number of times of emission for each of the plurality of electromagnetic waves on the basis of a result of the detection performed by the detector, the necessary number of times of emission being a certain number of times of emission that is necessary for measurement.

(2) The sensor apparatus according to (1), in which

the emitter emits each of the plurality of electromagnetic waves the necessary number of times of emission that is calculated by the calculator, and
the sensor apparatus further includes an averaging processor that generates an average electromagnetic wave by averaging electromagnetic waves for the necessary number of times of emission that are detected by the detector, the averaging being performed for each of the plurality of electromagnetic waves.

(3) The sensor apparatus according to (2), further including
a generator that generates target-object information regarding the measurement-target object on the basis of the average electromagnetic wave generated by the averaging processor.
(4) The sensor apparatus according to (2) or (3), in which

for each of the plurality of electromagnetic waves, the emitter emits a calculation-use electromagnetic wave used to calculate the necessary number of times of emission, and
the calculator calculates the necessary number of times of emission on the basis of a result of detection of the calculation-use electromagnetic wave that is performed by the detector.

(5) The sensor apparatus according to (4), in which
on the basis of a signal-to-noise ratio of the calculation-use electromagnetic wave detected by the detector, the calculator calculates, as the necessary number of times of emission, a certain number of times of emission with which a signal-to-noise ratio of the average electromagnetic wave generated by the averaging processor is greater than a specified threshold.
(6) The sensor apparatus according to (5), in which the calculator calculates the necessary number of times of emission using the following formula:

[Math. 1]

$$N = 10^{\frac{T - R}{10}}$$

, where N represents the necessary number of times of emission, T (dB) represents the specified threshold regarding the signal-to-noise ratio of the average electromagnetic wave, and R (dB) represents the signal-to-noise ratio of the calculation-use electromagnetic wave detected by the detector.
(7) The sensor apparatus according to (5) or (6), in which
the calculator calculates the necessary number of times of emission using a lookup table in which a correspondence relationship between the signal-to-noise ratio of the calculation-use electromagnetic wave detected by the detector and the necessary number of times of emission is defined.
(8) The sensor apparatus according to any one of (4) to (7), in which
the emitter emits the calculation-use electromagnetic waves in a frequency-based order with respect to the plurality of electromagnetic waves.
(9) The sensor apparatus according to any one of (4) to (7), in which
the emitter emits the calculation-use electromagnetic waves in a random order with respect to the plurality of electromagnetic waves.

(10) The sensor apparatus according to any one of (4) to (7), in which

the emitter controls an intensity of the calculation-use electromagnetic wave for each of the plurality of electromagnetic waves.

(11) The sensor apparatus according to any one of (5) to (7), in which

the specified thresholds regarding the signal-to-noise ratios of the average electromagnetic waves for the plurality of electromagnetic waves are set to an in-common value.

(12) The sensor apparatus according to any one of (5) to (7), in which

the specified threshold regarding the signal-to-noise ratio of the average electromagnetic wave is set for each of the plurality of electromagnetic waves.

(13) The sensor apparatus according to (12), in which

the specified threshold regarding the signal-to-noise ratio of the average electromagnetic wave is set for each of the plurality of electromagnetic waves such that the necessary number of times of emission that is calculated by the calculator is not greater than an upper limit.

(14) The sensor apparatus according to any one of (1) to (13), in which

with respect to each of the plurality of electromagnetic waves, the calculator sets an upper limit of the necessary number of times of emission, and calculates the necessary number of times of emission such that the necessary number of times of emission is not greater than the upper limit.

(15) The sensor apparatus according to any one of (1) to (14), in which

the calculator calculates the necessary numbers of times of emission of the plurality of electromagnetic waves of the frequencies on the basis of a result of detection of a portion of the plurality of electromagnetic waves that is selected on a frequency basis.

(16) The sensor apparatus according to (3), in which the generator calculates, as the target-object information, a moisture amount contained in the measurement-target object.

(17) The sensor apparatus according to (16), in which

the measurement-target object is soil, and
the generator calculates, as the target-object information, a moisture amount contained in the soil.

(18) An information processing apparatus, including:

an acquisition section that acquires a plurality of electromagnetic waves of different frequencies, each of the plurality of electromagnetic waves being emitted to a measurement-target object and detected by entering through the measurement-target object; and
a calculator that calculates a necessary number of times of emission for each of the plurality of electromagnetic waves acquired by the acquisition section, on the basis of each of the plurality of acquired electromagnetic waves, the necessary number of times of emission being a certain number of times of emission that is necessary for measurement.

(19) An information processing method that is performed by a computer system, the information processing method including:

acquiring a plurality of electromagnetic waves of different frequencies, each of the plurality of electromagnetic waves being emitted to a measurement-target object and detected by entering through the measurement-target object; and
calculating a necessary number of times of emission for each of the plurality of acquired electromagnetic waves on the basis of the electromagnetic wave, the necessary number of times of emission being a certain number of times of emission that is necessary for measurement.

(20) A program that causes a computer system to perform a process including:

acquiring a plurality of electromagnetic waves of different frequencies, each of the plurality of electromagnetic waves being emitted to a measurement-target object and detected by entering through the measurement-target object; and
calculating a necessary number of times of emission for each of the plurality of acquired electromagnetic waves on the basis of the electromagnetic wave, the necessary number of times of emission being a certain number of times of emission that is necessary for measurement.

Reference Signs List

1      sensor apparatus
4      information processing apparatus
5      transmission probe
6      reception probe
37     acquisition section
38     reception level calculator
39     number-of-times-of-emission calculator
40     transmission-and-reception controller
41     averaging processor
42     delay time calculator
43     relative permittivity calculator

44　moisture amount calculator

**Claims**

1. A sensor apparatus, comprising:

   an emitter that emits a plurality of electromagnetic waves of different frequencies to a measurement-target object;
   a detector that detects the plurality of electromagnetic waves entering the detector through the measurement-target object; and
   a calculator that calculates a necessary number of times of emission for each of the plurality of electromagnetic waves on a basis of a result of the detection performed by the detector, the necessary number of times of emission being a certain number of times of emission that is necessary for measurement.

2. The sensor apparatus according to claim 1, wherein

   the emitter emits each of the plurality of electromagnetic waves the necessary number of times of emission that is calculated by the calculator, and
   the sensor apparatus further comprises an averaging processor that generates an average electromagnetic wave by averaging electromagnetic waves for the necessary number of times of emission that are detected by the detector, the averaging being performed for each of the plurality of electromagnetic waves.

3. The sensor apparatus according to claim 2, further comprising
   a generator that generates target-object information regarding the measurement-target object on a basis of the average electromagnetic wave generated by the averaging processor.

4. The sensor apparatus according to claim 2, wherein

   for each of the plurality of electromagnetic waves, the emitter emits a calculation-use electromagnetic wave used to calculate the necessary number of times of emission, and
   the calculator calculates the necessary number of times of emission on a basis of a result of detection of the calculation-use electromagnetic wave that is performed by the detector.

5. The sensor apparatus according to claim 4, wherein
   on a basis of a signal-to-noise ratio of the calculation-use electromagnetic wave detected by the detector, the calculator calculates, as the necessary number of times of emission, a certain number of times of emission with which a signal-to-noise ratio of the average electromagnetic wave generated by the averaging processor is greater than a specified threshold.

6. The sensor apparatus according to claim **5,** wherein

   the calculator calculates the necessary number of times of emission using the following formula:

   [Math. 1]

   $$N = 10^{\frac{T-R}{10}},$$

   where N represents the necessary number of times of emission, T (dB) represents the specified threshold regarding the signal-to-noise ratio of the average electromagnetic wave, and R (dB) represents the signal-to-noise ratio of the calculation-use electromagnetic wave detected by the detector.

7. The sensor apparatus according to claim **5,** wherein
   the calculator calculates the necessary number of times of emission using a lookup table in which a correspondence relationship between the signal-to-noise ratio of the calculation-use electromagnetic wave detected by the detector and the necessary number of times of emission is defined.

8. The sensor apparatus according to claim **4,** wherein
   the emitter emits the calculation-use electromagnetic waves in a frequency-based order with respect to the plurality of electromagnetic waves.

9. The sensor apparatus according to claim **4,** wherein
   the emitter emits the calculation-use electromagnetic waves in a random order with respect to the plurality of electromagnetic waves.

10. The sensor apparatus according to claim **4,** wherein
    the emitter controls an intensity of the calculation-use electromagnetic wave for each of the plurality of electromagnetic waves.

11. The sensor apparatus according to claim **5,** wherein
    the specified thresholds regarding the signal-to-noise ratios of the average electromagnetic waves for the plurality of electromagnetic waves are set to an in-common value.

12. The sensor apparatus according to claim **5,** wherein
    the specified threshold regarding the signal-to-noise ratio of the average electromagnetic wave is set for each of the plurality of electromagnetic waves.

**13.** The sensor apparatus according to claim 12, wherein

the specified threshold regarding the signal-to-noise ratio of the average electromagnetic wave is set for each of the plurality of electromagnetic waves such that the necessary number of times of emission that is calculated by the calculator is not greater than an upper limit.

**14.** The sensor apparatus according to claim 1, wherein with respect to each of the plurality of electromagnetic waves, the calculator sets an upper limit of the necessary number of times of emission, and calculates the necessary number of times of emission such that the necessary number of times of emission is not greater than the upper limit.

**15.** The sensor apparatus according to claim 1, wherein the calculator calculates the necessary numbers of times of emission of the plurality of electromagnetic waves of the frequencies on a basis of a result of detection of a portion of the plurality of electromagnetic waves that is selected on a frequency basis.

**16.** The sensor apparatus according to claim 3, wherein the generator calculates, as the target-object information, a moisture amount contained in the measurement-target object.

**17.** The sensor apparatus according to claim 16, wherein

the measurement-target object is soil, and
the generator calculates, as the target-object information, a moisture amount contained in the soil.

**18.** An information processing apparatus, comprising:

an acquisition section that acquires a plurality of electromagnetic waves of different frequencies, each of the plurality of electromagnetic waves being emitted to a measurement-target object and detected by entering through the measurement-target object; and
a calculator that calculates a necessary number of times of emission for each of the plurality of electromagnetic waves acquired by the acquisition section, on a basis of each of the plurality of acquired electromagnetic waves, the necessary number of times of emission being a certain number of times of emission that is necessary for measurement.

**19.** An information processing method that is performed by a computer system, the information processing method comprising:

acquiring a plurality of electromagnetic waves of different frequencies, each of the plurality of electromagnetic waves being emitted to a measurement-target object and detected by entering through the measurement-target object; and
calculating a necessary number of times of emission for each of the plurality of acquired electromagnetic waves on a basis of the electromagnetic wave, the necessary number of times of emission being a certain number of times of emission that is necessary for measurement.

**20.** A program that causes a computer system to perform a process comprising:

acquiring a plurality of electromagnetic waves of different frequencies, each of the plurality of electromagnetic waves being emitted to a measurement-target object and detected by entering through the measurement-target object; and
calculating a necessary number of times of emission for each of the plurality of acquired electromagnetic waves on a basis of the electromagnetic wave, the necessary number of times of emission being a certain number of times of emission that is necessary for measurement.

FIG.1

Start

Frequency f = fstart — ST101

Perform transmission with prescribed level with respect to frequency f — ST102

Perform reception and storing — ST103

Detect reception level — ST104

Calculate necessary number N of times of emission — ST105

Perform process of transmission-and-reception routine — ST106

Average reception signals for N times — ST107

$f = f + fstep$ — ST108

$f > fend$ ? — ST109

No

Yes

End

FIG.2

```
                    ┌──────────────┐
                    │    Start     │
                    └──────┬───────┘
                           │
                           ▼
      ┌────────────────────────────────────────┐
      │                 n = 1                   │──── ST201
      └────────────────────┬───────────────────┘
                           │         ◄──────────────────┐
                           ▼                            │
                    ╱─────────────╲                     │
           Yes    ╱    n = N ?      ╲──── ST202          │
      ◄──────────╲                 ╱                     │
                   ╲─────────────╱                       │
                           │ No                          │
                           ▼                             │
      ┌────────────────────────────────────────┐        │
      │ Perform transmission with prescribed    │──── ST203
      │ level with respect to frequency f       │        │
      └────────────────────┬───────────────────┘        │
                           ▼                             │
      ┌────────────────────────────────────────┐        │
      │     Perform reception and storing       │──── ST204
      └────────────────────┬───────────────────┘        │
                           ▼                             │
      ┌────────────────────────────────────────┐        │
      │               n = n + 1                 │──── ST205
      └────────────────────┬───────────────────┘        │
                           └─────────────────────────────┘
                           │
                           ▼
                    ┌──────────────┐
                    │     End      │
                    └──────────────┘
```

# FIG.3

A Level of transmission signal

Sweep

Frequency

B Level of reception signal

Frequency

C SNR

Necessary SNR

Frequency

D Necessary number of times of emission (Average number of times)

Frequency

E SNR

Necessary SNR

Frequency

FIG.4

FIG.5

FIG.6

FIG.7

FIG.8

FIG.9

FIG.10

```
          ┌──────────┐
          │  Start   │
          └────┬─────┘
               ▼
     ┌───────────────────┐
     │      n = 1        │────  ST401
     └─────────┬─────────┘
               ▼
         ◇ n = N(f) ? ◇ ──── ST402
    Yes ╱           ╲ No
```

Start

n = 1                                      ST401

n = N(f) ?                                 ST402

Yes

No

Perform transmission with prescribed
level with respect to frequency f          ST403

Perform reception and storing             ST404

n = n + 1                                  ST405

End

FIG.11

FIG.12

# INTERNATIONAL SEARCH REPORT

| | International application No. |
|---|---|
| | **PCT/JP2023/011413** |

## A. CLASSIFICATION OF SUBJECT MATTER

***G01R 29/08***(2006.01)i; ***G01N 22/00***(2006.01)i; ***G01N 22/04***(2006.01)i
FI:   G01N22/04 Z; G01R29/08 F; G01N22/00 Y; G01N22/00 N

According to International Patent Classification (IPC) or to both national classification and IPC

## B. FIELDS SEARCHED

Minimum documentation searched (classification system followed by classification symbols)

G01N22/00-G01N22/04, G01R29/00-G01R29/26, G01R23/00-G01R23/20, G01R27/00-G01R27/32, G01N21/3581, G01N21/3586, A61B5/05

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580/JSTChina (JDreamIII), Science Direct, IEEE Xplore, APS Journals

## C. DOCUMENTS CONSIDERED TO BE RELEVANT

| Category* | Citation of document, with indication, where appropriate, of the relevant passages | Relevant to claim No. |
|---|---|---|
| A | US 2021/0262946 A1 (VAYYAR IMAGING LTD) 26 August 2021 (2021-08-26) claims 1-20 | 1-20 |
| A | US 2013/0332115 A1 (UNIVERSITY OF NOTRE DAME DU LAC) 12 December 2013 (2013-12-12) | 1-20 |
| A | US 7330034 B1 (THE UNITED STATES OF AMERICA AS REPRESENTED BY THE SECRETARY OF AGRICULTURE) 12 February 2008 (2008-02-12) | 1-20 |
| A | US 2018/0020948 A1 (MASSACHUSETTS INSTITUTE OF TECHNOLOGY) 25 January 2018 (2018-01-25) | 1-20 |
| A | WO 2002/031479 A1 (NEW ZEALAND FOREST RESEARCH INSTITUTE LIMITED) 18 April 2002 (2002-04-18) | 1-20 |
| A | WO 2021/241723 A1 (HYOGO COLLEGE MEDICINE) 02 December 2021 (2021-12-02) | 1-20 |
| A | JP 2001-245865 A (SEKISUI CHEM CO LTD) 11 September 2001 (2001-09-11) | 1-20 |
| A | JP 2019-190895 A (NIPPON TELEGRAPH & TELEPHONE) 31 October 2019 (2019-10-31) | 1-20 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 May 2023** | **30 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/011413** |

**C. DOCUMENTS CONSIDERED TO BE RELEVANT**

| Category* | Citation of document, with indication, where appropriate, of the relevant passages | Relevant to claim No. |
| --- | --- | --- |
| A | JP 2019-190896 A (NIPPON TELEGRAPH & TELEPHONE) 31 October 2019 (2019-10-31) | 1-20 |
| A | JP 2010-101789 A (OJI PAPER CO LTD) 06 May 2010 (2010-05-06) | 1-20 |
| A | JP 2006-349425 A (OJI PAPER CO LTD) 28 December 2006 (2006-12-28) | 1-20 |
| A | WO 2011/092889 A1 (OJI PAPER CO LTD) 04 August 2011 (2011-08-04) | 1-20 |
| A | WO 2015/064370 A1 (OJI HOLDINGS CO LTD) 07 May 2015 (2015-05-07) | 1-20 |

Form PCT/ISA/210 (second sheet) (January 2015)

**INTERNATIONAL SEARCH REPORT**
Information on patent family members

International application No.

**PCT/JP2023/011413**

| Patent document cited in search report | | | Publication date (day/month/year) | Patent family member(s) | | | Publication date (day/month/year) |
|---|---|---|---|---|---|---|---|
| US | 2021/0262946 | A1 | 26 August 2021 | US | 2018/0224382 | A1 | |
| | | | | WO | 2017/021950 | A2 | |
| US | 2013/0332115 | A1 | 12 December 2013 | US | 9037414 | B1 | |
| US | 7330034 | B1 | 12 February 2008 | (Family: none) | | | |
| US | 2018/0020948 | A1 | 25 January 2018 | WO | 2016/126999 | A1 | |
| WO | 2002/031479 | A1 | 18 April 2002 | US | 2004/0124855 | A1 | |
| | | | | AU | 1285102 | A1 | |
| | | | | CA | 2424214 | A1 | |
| | | | | NZ | 525035 | A | |
| | | | | AU | 2002212851 | B2 | |
| WO | 2021/241723 | A1 | 02 December 2021 | (Family: none) | | | |
| JP | 2001-245865 | A | 11 September 2001 | (Family: none) | | | |
| JP | 2019-190895 | A | 31 October 2019 | US | 2021/0177310 | A1 | |
| | | | | WO | 2019/203110 | A1 | |
| JP | 2019-190896 | A | 31 October 2019 | US | 2021/0161419 | A1 | |
| | | | | WO | 2019/203153 | A1 | |
| JP | 2010-101789 | A | 06 May 2010 | (Family: none) | | | |
| JP | 2006-349425 | A | 28 December 2006 | US | 2006/0288782 | A1 | |
| | | | | EP | 1734361 | A1 | |
| | | | | DE | 602006000507 | T2 | |
| | | | | CN | 1880947 | A | |
| | | | | CA | 2550164 | A1 | |
| WO | 2011/092889 | A1 | 04 August 2011 | US | 2013/0025350 | A1 | |
| | | | | EP | 2530458 | A1 | |
| | | | | CA | 2793047 | A1 | |
| | | | | CN | 102782484 | A | |
| WO | 2015/064370 | A1 | 07 May 2015 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018221051 A